# EUROPEAN PATENT APPLICATION

(11) **EP 4 614 093 A2**
(43) Date of publication of application: **10.09.2025**
(21) Application number: 24218500.7
(22) Date of filing: 09.12.2024
(51) Int. Cl.: F25J 3/06, B01D 53/14, F25J 1/00, B01D 53/04, B01J 20/34, C01B 32/50

(54) **SYSTEM AND METHOD FOR CAPTURING CARBON DIOXIDE FROM AN AIR STREAM**

(30) Priority: 15.12.2023 US 202363610466 P
(71) Applicant: Carrier Corporation, Palm Beach Gardens, FL 33418 (US)
(72) Inventor: GRABON, Michel, Bressolles (FR)
(74) Representative: Dehns

(57) **Abstract**

Described herein is a system (100) for capturing carbon dioxide (CO₂) from an air stream. The system comprises a CO₂ capturing media (102) configured to allow the air stream to flow therethrough and correspondingly capture CO₂ gas present in the air stream, and a liquefication unit configured to receive the captured CO₂ gas from the CO₂ capturing media (102) and correspondingly compress and/or liquefy the received CO₂ gas into liquid CO₂, wherein heat generated during the compression and/or liquefication of the CO₂ is supplied to the CO₂ capturing media (102) to regenerate the CO₂ capturing media (102).

## Description

### BACKGROUND

This invention relates to an improved and efficient system and method for capturing carbon dioxide (CO₂) or hydrocarbon gases from an air stream.

### SUMMARY

According to a first aspect of the invention there is provided a system for capturing carbon dioxide (CO₂) from an air stream. The system comprises a CO₂ capturing media configured to allow the air stream to flow therethrough and correspondingly capture CO₂ gas present in the air stream, and a liquefication unit configured to receive the captured CO₂ gas from the CO₂ capturing media and correspondingly compress and/or liquefy the received CO₂ gas into liquid CO₂, wherein heat generated during the compression and/or liquefication of the CO₂ is supplied to the CO₂ capturing media to regenerate the CO₂ capturing media.

Optionally, the liquefication unit comprises a compressor configured to receive the captured CO₂ gas from the CO₂ capturing media and correspondingly compress the received CO₂ gas, wherein the compression of received CO₂ gas results in heating of the CO₂ gas, and a conduit fluidically connected to an outlet of the compressor, the conduit in thermal contact and extending in a predefined configuration through the CO₂ capturing media, wherein the heated compressed CO₂ gas is supplied through the conduit to heat the CO₂ capturing media and correspondingly regenerate the CO₂ capturing media and liquefy the compressed CO₂ gas into liquid CO₂.

Optionally, the liquefication unit comprises a compressor configured to receive the captured CO₂ gas from the CO₂ capturing media and correspondingly compress the received CO₂ gas, a heat exchanger fluidically connected to an outlet of the compressor, the heat exchanger configured to allow flow of the compressed CO₂ gas therethrough, and wherein the heat exchanger is further configured to allow flow of air across the heat exchanger to facilitate heat exchange between the air and the compressed CO₂ gas, wherein the heat exchange causes the air to heat and liquefy the compressed CO₂ gas into liquid CO₂, wherein the heated air is supplied to the CO₂ capturing media to regenerate the CO₂ capturing media.

Optionally, the system comprises one or more fans to facilitate the flow of the air across the heat exchanger and further supply the heated air to the CO₂ capturing media.

Optionally, the system comprises a storage container fluidically connected to the liquefication unit and configured to receive and store the generated liquid CO₂.

Optionally, the CO₂ capturing media is selected from a group comprising a CO₂ adsorption bed, and a CO₂ absorption bed.

Optionally, the CO₂ capturing media is positioned in a passage or containment space that is configured to be fluidically isolated from ambient to allow the captured CO₂ to dissolve therewithin and be further collected by the liquefication unit, wherein an inlet of the liquefication unit is blocked during dissolving of the captured.

Optionally, the system comprises one or more gas sensors configured with the CO₂ capturing media or position downstream of the CO₂ capturing media, the one or more gas sensors are configured to monitor an amount of CO₂ captured by the CO₂ capturing media.

Optionally, when the monitored amount of CO₂ captured by the CO₂ capturing media exceeds a predefined saturation level, the system is configured to actuate the liquefication unit to receive the captured CO₂ gas from the CO₂ capturing media to compress or liquefy the CO₂ gas, and supply the heat generated during the compression or liquefication to the CO₂ capturing media for regeneration of CO₂ capturing media.

According to a second aspect of the invention there is provided a method for capturing carbon dioxide (CO₂) from an air stream. The method comprises allowing the air stream to flow through a CO₂ capturing media to capture CO₂ gas present in the air stream, compressing and/or liquefying the captured CO₂ gas into liquid CO₂, and supplying heat generated during the compression and/or liquefication of the CO₂ to the CO₂ capturing media for regeneration of the CO₂ capturing media.

Optionally, the method comprises receiving, by a compressor, the captured CO₂ gas from the CO₂ capturing media and correspondingly compressing the received CO₂ gas, wherein the compression of received CO₂ gas results in heating of the compressed CO₂ gas, and supplying, by a conduit extending in a predefined configuration through the CO₂ capturing media, the heated compressed CO₂ gas through the CO₂ capturing media to regenerate the CO₂ capturing media and correspondingly liquefy the compressed CO₂ gas into liquid CO₂.

Optionally, the method comprises receiving, by a compressor, the captured CO₂ gas from the CO₂ capturing media and correspondingly compressing the received CO₂ gas, allowing flow of the compressed CO₂ gas through a heat exchanger fluidically connected to an outlet of the compressor, allowing flow of air across the heat exchanger to facilitate heat exchange between the air and the compressed CO₂ gas, wherein the heat exchange causes the air to be heated and the compressed CO₂ gas to liquefy into liquid CO₂, and supplying the heated air through the CO₂ capturing media for regeneration of the CO₂ capturing media.

Optionally, the method comprises the steps of supplying and storing the generated liquid CO₂ in a container.

Optionally, the CO₂ capturing media is positioned in a passage or containment space that is configured to be fluidically isolated from ambient to allow the captured CO₂ to dissolve therewithin and be further collected by the compressor, wherein an inlet of the compressor is blocked during dissolving of the captured CO₂.

Optionally, the method comprises the steps of monitoring, by one or more gas sensors, an amount of CO₂ captured by the CO₂ capturing media, and when the monitored amount of CO₂ captured by the CO₂ capturing media exceeds a predefined saturation level, the method comprises the steps of receiving, by a liquefication unit, the captured CO₂ gas from the CO₂ capturing media to compress and/or liquefy the CO₂ gas, and supplying the heat generated during the compression and/or liquefication to the CO₂ capturing media for regeneration of CO₂ capturing media.

According to a third aspect of the invention there is provided a method for capturing hydrocarbon gases from an air stream. The method comprises allowing the air stream to flow through a hydrocarbon capturing media to capture hydrocarbon gases present in the air stream, compressing or liquefying, by a liquefication unit, the captured hydrocarbon gases into liquid hydrocarbon, and supplying heat generated during the compression or liquefication of the hydrocarbon gases to the hydrocarbon capturing media for regeneration of the hydrocarbon capturing media

Optionally, the method comprises receiving, by a compressor, the captured hydrocarbon gases from the hydrocarbon capturing media and correspondingly compressing the received hydrocarbon gases, wherein the compression of the hydrocarbon gases results in heating of the hydrocarbon gases, and supplying, by a conduit extending in a predefined configuration through the hydrocarbon capturing media, the heated compressed hydrocarbon gases through the hydrocarbon capturing media to regenerate the hydrocarbon capturing media and correspondingly liquefy the compressed hydrocarbon gases into liquid hydrocarbon.

Optionally, the method comprises receiving, by a compressor, the captured hydrocarbon gases from the hydrocarbon capturing media and correspondingly compressing the received hydrocarbon gases, allowing flow of the compressed hydrocarbon gases through a heat exchanger fluidically connected to an outlet of the compressor; allowing flow of air across the heat exchanger to facilitate heat exchange between the air and the compressed hydrocarbon gases, wherein the heat exchange causes the air to be heated and the compressed hydrocarbon gases to liquefy into liquid hydrocarbon, and supplying the heat air through the hydrocarbon capturing media for regeneration of the hydrocarbon capturing media.

Optionally, the method comprises the steps of supplying and storing the generated liquid hydrocarbon in a container, wherein the hydrocarbon gases comprise methane, propane, and/or ethane.

Optionally, the method comprises the steps of monitoring, by one or more gas sensors, an amount of the hydrocarbon gases captured by the hydrocarbon capturing media, and when the monitored amount of hydrocarbon gases captured by the hydrocarbon capturing media exceeds a predefined saturation level, the method comprises the steps of receiving, by a liquefication unit, the captured hydrocarbon gases from the hydrocarbon capturing media to compress and/or liquefy the hydrocarbon gases, and supplying the heat generated during the compression or liquefication to the hydrocarbon capturing media for regeneration of hydrocarbon capturing media.

The foregoing summary is illustrative only and is not intended to be in any way limiting. In addition to the illustrative aspects, embodiments, and features described above, further aspects, embodiments, features, and techniques of the invention will become more apparent from the following description taken in conjunction with the drawings.

### BRIEF DESCRIPTION OF THE DRAWINGS

The accompanying drawings are included to provide a further understanding of the invention and are incorporated in and constitute a part of this specification. The drawings illustrate exemplary embodiments of the invention and, together with the description, serve to explain the principles of the invention.

In the drawings, similar components and/or features may have the same reference label. Further, various components of the same type may be distinguished by following the reference label with a second label that distinguishes among the similar components. If only the first reference label is used in the specification, the description is applicable to any one of the similar components having the same first reference label irrespective of the second reference label.
FIG. 1 illustrates a system for capturing carbon dioxide from an airstream.
FIG. 2 illustrates another system for capturing carbon dioxide from an airstream.
FIG. 3 illustrates an exemplary flow diagram depicting the method for capturing carbon dioxide from an airstream.
FIG. 4 illustrates an exemplary flow diagram depicting the method for capturing hydrocarbon gases from an airstream.

### DETAILED DESCRIPTION

The following is a detailed description of embodiments of the invention depicted in the accompanying drawings. The embodiments are in such detail as to clearly communicate the invention. However, the amount of detail offered is not intended to limit the anticipated variations of embodiments; on the contrary, the intention is to cover all modifications, equivalents, and alternatives falling within the scope of the invention as defined by the appended claims.

Various terms are used herein. To the extent a term used in a claim is not defined below, it should be given the broadest definition persons in the pertinent art have given that term as reflected in printed publications and issued patents at the time of filing.

In the specification, reference may be made to the spatial relationships between various components and to the spatial orientation of various aspects of components as the devices are depicted in the attached drawings. However, as will be recognized by those skilled in the art after a complete reading of the subject disclosure, the components of this invention described herein may be positioned in any desired orientation. Thus, the use of terms such as "above," "below," "upper," "lower," "first", "second" or other like terms to describe a spatial relationship between various components or to describe the spatial orientation of aspects of such components should be understood to describe a relative relationship between the components or a spatial orientation of aspects of such components, respectively, described herein may be oriented in any desired direction.

In conventional carbon dioxide (CO₂) capturing processes, adsorption or absorption beds (CO₂ capturing media) serve as pivotal components for removing CO₂ from air streams. During exposure to air with elevated CO₂ content, the bed effectively absorbs or adsorbs CO₂, facilitating an air purification process. However, this process halts when the bed reaches saturation with CO₂.

To resume CO₂ capture, the bed must undergo a regeneration process. Typically, heat is applied to the bed during regeneration, causing the release of CO₂. The liberated CO₂ is then collected and subjected to liquefication or solidification for subsequent storage or reuse.

The challenge inherent in this conventional approach lies in the substantial energy requirements associated with both bed regeneration and CO₂ liquefication processes. Bed regeneration, achieved through heating, demands a significant amount of thermal energy, with heating temperatures typically reaching around 100 °C to effectively release and remove CO₂ from the bed. Meanwhile, the CO₂ liquefication or solidification process involves compressing CO₂ and subsequently cooling it to induce a phase change from gas to liquid, necessitating high-pressure conditions.

As a result of the energy-intensive nature of bed regeneration and CO₂ liquefication, there is a need for a solution to enhance the overall efficiency of CO₂ capture processes. Specifically, there is a need to explore sustainable systems and methods for utilizing the heat generated during the CO₂ liquefication process to optimize the bed regeneration step. Such integration has the potential to substantially reduce the external energy requirements for both processes, leading to significant energy savings.

Embodiments of the invention (system and method) address the above-mentioned challenges by providing an efficient and economically viable solution that harnesses the rejected heat during CO₂ compression to enhance the bed regeneration process.

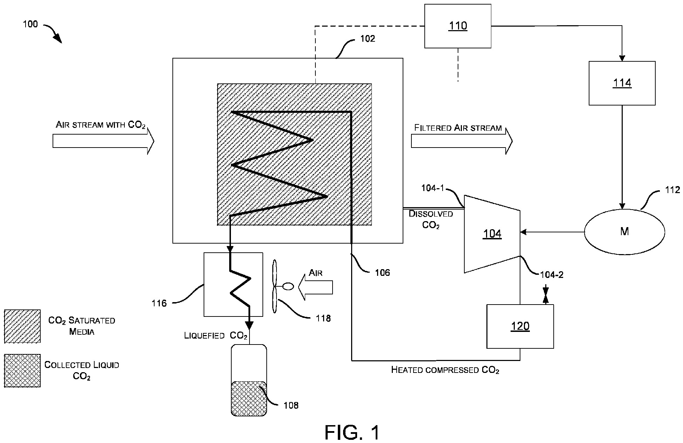

Referring to FIGs. 1 to 2, a system 100 for capturing carbon dioxide (CO₂) from an air stream is disclosed. The system 100 can include a CO₂ capturing media 102 configured to allow the air stream to flow therethrough and correspondingly capture CO₂ gas present in the air stream. In one or more embodiments, the CO₂ capturing media 102 can be selected from a group comprising a CO₂ adsorption bed, and a CO₂ absorption bed, but is not limited to the like.

The CO₂ capturing media 102 can be configured (provided) in an air purification or conditioning unit such that the CO₂ capturing media 102 remains in a flow path of the air stream to be purified or filtered. The system 100 can further include a liquefication unit that can be configured to receive the captured CO₂ gas from the CO₂ capturing media 102 and correspondingly compress and/or liquefy the received CO₂ gas into liquid CO₂. This compression of the CO₂ gas can result in the generation of heat or raising the temperature of the CO₂ gas in a range of 80 to 100°C. Accordingly, the heat generated during the compression or liquefication of the CO₂ can then be used or supplied to the CO₂ capturing media 102 to regenerate the CO₂ capturing media 102, thereby renewing the CO₂ capturing media 102 for further CO₂ capturing.

In one or more embodiments, the CO₂ capturing media 102 can be positioned in a passage or containment space in the air purification or conditioning unit. The passage or the containment space can be configured to allow the air stream to flow through the CO₂ capturing media 102 during the CO₂ capturing process. However, during CO₂ collection process or CO₂ capturing media 102 regeneration process, the passage or the containment space can be configured to be fluidically isolated from ambient or rest of the air purification or conditioning unit, which allows the captured CO₂ to dissolve from the CO₂ capturing media 102 and be collected in the passage or containment space without getting affected by the air stream or external environment. During this dissolving process, an inlet of the liquefication unit can also be kept closed or blocked to facilitate in collection of the dissolved CO₂ within the passage or containment space. Later, the dissolved CO₂ can be supplied to or received by the liquefication unit for further compression or liquefication. Furthermore, the system 100 can include a storage container 108 fluidically connected to the liquefication unit and configured to receive and store the generated liquid CO₂.

In one or more embodiments, the system 100 can include one or more gas sensors 110 configured with the CO₂ capturing media 102 or positioned downstream of the CO₂ capturing media 102. The gas sensors 110 can be configured to monitor an amount of CO₂ captured by the CO₂ capturing media 102 by monitoring the CO₂ content in the air stream downstream of the CO₂ capturing media 102, however, in other embodiments, the gas sensors 110 can be configured directly with the CO₂ capturing media 102 to directly monitor the amount of CO₂ captured by the CO₂ capturing media 102.

In one or more embodiments, when the monitored amount of CO₂ captured by the CO₂ capturing media 102 exceeds a predefined saturation level, the system 100 can be configured to actuate the liquefication unit to receive the captured CO₂ gas from the CO₂ capturing media 102 and correspondingly compress and/or liquefy the CO₂ gas and further supply the heat generated during the compression or liquefication to the CO₂ capturing media 102 for regeneration of CO₂ capturing media 102. This may enable the system 100 to continuously capture CO₂ from the air stream and parallelly regenerate the CO₂ capturing media 102 without affecting the overall CO₂ capturing process.

Referring to FIG. 1, in one or more embodiments, the liquefication unit can include a compressor 104 having an inlet 104-1 configured to receive the captured CO₂ gas from the CO₂ capturing media 102 and correspondingly compress the received CO₂ gas. It should be obvious to a person skilled in the art that the compression of the CO₂ gas by the compressor 104 can result in heating the compressed CO₂ gas to a temperature in a range of 80 to 100°C. The compressor 104 may be a variable-speed compressor 104 that can be driven by a variable-speed motor 112. However, the compressor 104 may be a fixed-speed compressor 104 that can be driven by a fixed-speed motor. Further, a conduit 106 can be fluidically connected to an outlet 104-2 of the compressor 104 via a valve (not shown). The conduit 106 can extend in a predefined configuration through the CO₂ capturing media 102 such that at least a section of the conduit 106 remains in thermal contact with the CO₂ capturing media 102. Accordingly, when the heated compressed CO₂ gas is supplied through the conduit 106, the heated compressed CO₂ gas can heat the CO₂ capturing media 102 and correspondingly regenerate the CO₂ capturing media 102. This heat exchange between the CO₂ capturing media 102 and the heated compressed CO₂ gas during the regeneration process can result in the liquefication of the compressed CO₂ gas into liquid CO₂ and further lower the temperature of the liquid CO₂. Further, in one or more embodiments, an additional air/CO₂ heat exchanger 116 can be configured downstream of an outlet of the conduit 106 (extending out of the CO₂ capturing media 102) or upstream of the liquid storage container 108 to facilitate liquefication of the CO₂ if the heat absorbed by desorption process while flowing through CO₂ capturing media 102 is smaller than the CO₂ liquefication cooling process. Further, an additional fan 118 can be configured with the heat exchanger 116 to enhance the heat exchange by flowing external air across the heat exchanger 116.

In one or more embodiments, the liquefication unit can include a gas separator 120 configured at outlet 104-2 of the compressor 104. The gas separator 120 may be a reservoir configured with a venting system. During the regeneration process, the volume of CO₂ (hydrocarbon) capturing media 102 can be full of air and the media (bed) 102 can be saturated with CO₂ (hydrocarbon). Further, when the media 102 regeneration process starts, the media 102 can be insulated from the air intake with the compressor pulling air from the media 102. Later, as the pressure of the air goes down, media 102 starts to release CO₂. During this pull-down process, the composition of a mixture of air/CO₂ (hydrocarbon) can change from "poor CO2" (high air concentration) to rich CO₂ (low air concentration). As a result, at the compressor discharge (outlet 104-2) there may be a need to separate the air (non-condensable) from CO₂ (hydrocarbon). Accordingly, from time to time (when the pressure of the mixture of air/CO₂ (hydrocarbon) is too high), the gas separator 120 can vent air, using the venting system, from the top of the reservoir associated with the gas separator 120 and supply "pure" CO₂ (hydrocarbon) from the bottom to push it into the conduit 106 for further desorption/liquefication process.

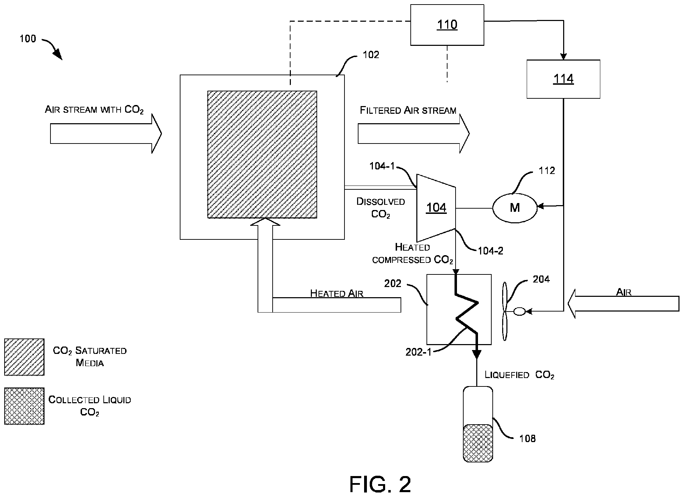

Referring to FIG. 2, in one or more embodiments, the liquefication unit can include a compressor 104 having an inlet 1024-1 configured to receive the captured CO₂ gas from the CO₂ capturing media 102 and correspondingly compress the received CO₂ gas, resulting in heating of the compressed CO₂ gas to a temperature in a range of 80 to 100°C. The liquefication unit can further include a heat exchanger 202 having a coil 202-1 fluidically connected to an outlet 104-2 of the compressor 104 via another valve (not shown). The heat exchanger coil 202-1 can be configured to receive and allow the flow of the heated compressed CO₂ gas therethrough. The heat exchanger 202 can be further configured to allow the flow of air across the heat exchanger 202 to facilitate heat exchange between the air and the heated CO₂ gas. This heat exchange can cause the air to be heated and further liquefy the compressed CO₂ gas into liquid CO₂ and lowering the temperature of the liquid CO₂. Further, the heated air can then be supplied to the CO₂ capturing media 102 to regenerate the CO₂ capturing media 102. In one or more embodiments, the system 100 can include one or more fans 204 to facilitate the flow of the air across the heat exchanger 202 and further supply the heated air to the CO₂ capturing media 102.

In one or more embodiments, the system 100 can include a controller 114 in communication with the compressor 104 (compressor 104 motor), the heat exchanger 202, the fans 204, the gas sensors 110, and valve(s). The controller 114 can monitor, using the gas sensors 110, the amount of CO₂ captured by the CO₂ capturing media 102. Further, when the captured CO₂ level exceeds a predefined saturation level, the controller 114 can actuate the compressor 104 to receive the captured CO₂ gas from the CO₂ capturing media 102 and correspondingly compress the CO₂ gas, which may heat the CO₂ gas.

In one or more embodiments, referring to FIG. 1, the controller 114 can actuate the valve configured in the conduit 106 extending through the CO₂ capturing media 102, to supply the heated CO₂ gas through the CO₂ capturing media 102 for regeneration of CO₂ capturing media 102, which may further liquefy and cool the CO₂.

Further, in one or more embodiments, referring to FIG. 2, the controller 114 can actuate the compressor 104 and the valve to supply the compressed heated CO₂ gas through the heat exchanger coils 202 and further actuate the fans 204 to supply air across the heat exchanger 202, thereby facilitating heat exchange between the air and the heated CO₂ gas. This heat exchange can cause the air to be heated and further liquefy the compressed CO₂ gas into liquid CO₂ and lowering the temperature of the liquid CO₂. Further, the fans 204 can be actuated to supply the heated air to the CO₂ capturing media 102 to regenerate the CO₂ capturing media 102.

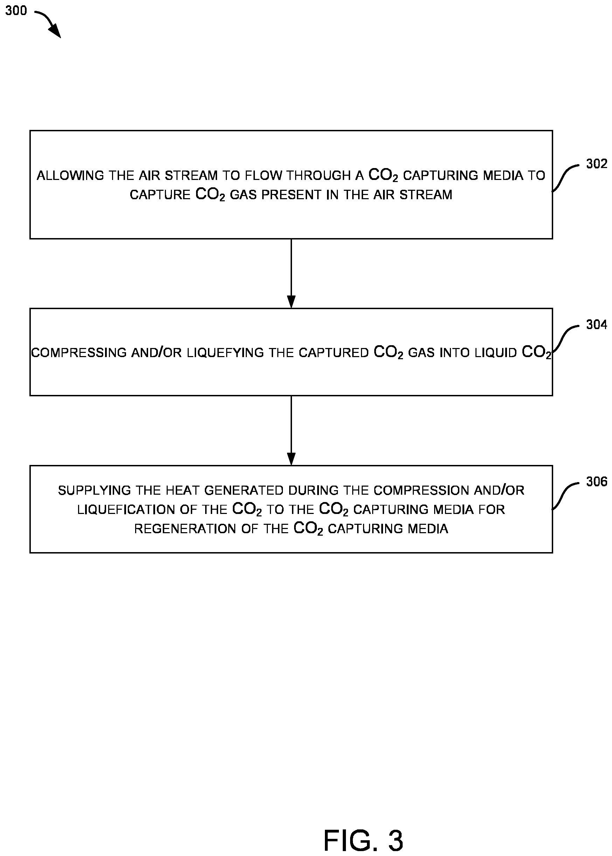

Referring to FIG. 3, a method 300 for capturing carbon dioxide (CO₂) from an air stream is disclosed. The method 300 can include the CO₂ capturing media 102, the compressor 104, the controller 114, and other components associated with the system 100 of FIGs. 1 and 2. The method 300 can include step 302 of allowing the air stream to flow through a CO₂ capturing media to capture CO₂ gas present in the air stream, followed by another step 304 of compressing and/or liquefying the captured CO₂ gas into liquid CO₂. Further, the method 300 can include step 306 of supplying the heat generated during the compression and/or liquefication of the CO₂ to the CO₂ capturing media for regeneration of the CO₂ capturing media.

In one or more embodiments, at step 304, a compressor can receive the captured CO₂ gas from the CO₂ capturing media and correspondingly compress the received CO₂ gas. This compression of received CO₂ gas can result in the heating of the compressed CO₂ gas. Further, at step 306, a conduit extending in a predefined configuration through the CO₂ capturing media can supply the heated compressed CO₂ gas through the CO₂ capturing media to regenerate the CO₂ capturing media and correspondingly liquefy the compressed CO₂ gas into liquid CO₂.

In one or more embodiments, at step 304, a compressor can receive the captured CO₂ gas from the CO₂ capturing media and correspondingly compress the received CO₂ gas. This compression of received CO₂ gas can result in the heating of the compressed CO₂ gas. Further, at step 304, the heated compressed CO₂ gas can be supplied through a heat exchanger that is fluidically connected to an outlet of the compressor. Furthermore, at step 306, the heat exchanger can further allow the flow of air across to facilitate heat exchange between the air and the compressed CO₂ gas, which may cause the air to be heated and the compressed CO₂ gas to liquefy into liquid CO₂. Later, at step 306, the heated air can be supplied through the CO2 capturing media to regenerate the CO₂ capturing media.

In one or more embodiments, the method 300 can further include the steps of monitoring, by one or more gas sensors, an amount of the CO₂ gas captured by the CO₂ capturing media. Accordingly, when the monitored amount of CO₂ gas captured by the CO₂ capturing media exceeds a predefined saturation level, the method 300 can include the steps of receiving, by a liquefication unit, the captured CO₂ gas from the CO₂ capturing media to compress and/or liquefy the CO₂ gas, followed by another step of supplying the heat generated during the compression or liquefication to the CO₂ capturing media for regeneration of the CO₂ capturing media. The steps of method 300 can be parallelly executed to enable the continuous capturing of CO₂ from the air stream and parallelly regenerate the CO₂ capturing media without affecting the overall CO₂ capturing process.

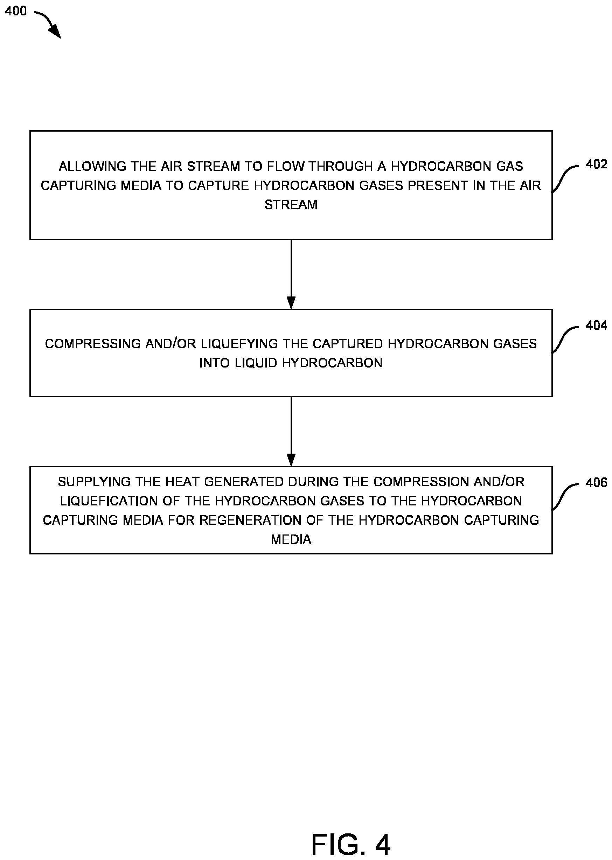

Referring to FIG. 4, a method 400 for capturing hydrocarbon gases such as but not limited to ethane, propane, methane, and the like from an air stream is disclosed. The method 400 can include step 402 of allowing the air stream to flow through a hydrocarbon gas capturing media to capture hydrocarbon gases present in the air stream, followed by another step 404 of compressing and/or liquefying the captured hydrocarbon gases into liquid hydrocarbon. Further, the method 400 can include step 406 of supplying the heat generated during the compression and/or liquefication of the hydrocarbon gases to the hydrocarbon capturing media for regeneration of the hydrocarbon capturing media.

In one or more embodiments, at step 404, a compressor can receive the captured hydrocarbon gases from the hydrocarbon capturing media and correspondingly compress the received hydrocarbon gases. This compression of received hydrocarbon gases can result in the heating of the compressed hydrocarbon gases. Further, at step 406, a conduit extending in a predefined configuration through the hydrocarbon capturing media can supply the heated compressed hydrocarbon gases through the hydrocarbon capturing media to regenerate the hydrocarbon capturing media and correspondingly liquefy the compressed hydrocarbon gases into liquid hydrocarbon.

In one or more embodiments, at step 404, a compressor can receive the captured hydrocarbon gases from the hydrocarbon capturing media and correspondingly compress the received hydrocarbon gases. This compression of received hydrocarbon gases can result in the heating of the compressed hydrocarbon gases. Further, at step 404, a conduit can supply the heated compressed hydrocarbon gases through a heat exchanger fluidically connected to an outlet of the compressor via the conduit. Furthermore, at step 406, the heat exchanger can further allow the flow of air across to facilitate heat exchange between the air and the compressed hydrocarbon gases, which may cause the air to be heated and the compressed hydrocarbon gases to liquefy into liquid hydrocarbon. In addition, at step 406, the heated air can be supplied through the hydrocarbon capturing media to regenerate the hydrocarbon capturing media.

In one or more embodiments, the method 400 can further include the steps of monitoring, by one or more gas sensors, an amount of the hydrocarbon gases captured by the hydrocarbon capturing media. Accordingly, when the monitored amount of hydrocarbon gases captured by the hydrocarbon capturing media exceeds a predefined saturation level, the method 400 can include the steps of receiving, by a liquefication unit, the captured hydrocarbon gases from the hydrocarbon capturing media to compress and/or liquefy the hydrocarbon gases, followed by another step of supplying the heat generated during the compression or liquefication to the hydrocarbon capturing media for regeneration of the hydrocarbon capturing media. The steps of method 400 can be parallelly executed to enable the continuous capturing of hydrocarbon gas from the air stream and parallelly regenerate the hydrocarbon capturing media without affecting the overall hydrocarbon capturing process.

Thus, embodiments of this invention overcome the drawbacks, limitations, and shortcomings associated with existing CO₂ (and hydrocarbon gas) capturing and regeneration processes, by providing an efficient and economically viable system and method that harnesses the rejected heat during CO₂ (or hydrocarbon gas) compression to enhance the capturing media regeneration process.

While the invention has been described with reference to exemplary embodiments, it will be understood by those skilled in the art that various changes may be made and equivalents may be substituted for elements thereof without departing from the scope of the invention as defined by the appended claims. Modifications may be made to adopt a particular situation or material to the teachings of the invention without departing from the scope thereof. Therefore, it is intended that the invention not be limited to the particular embodiments disclosed, but that the invention includes all embodiments falling within the scope of the invention as defined by the appended claims.

In interpreting the specification, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refer to at least one of something selected from the group consisting of A, B, C ....and N, the text should be interpreted as requiring only one element from the group, not A plus N, or B plus N, etc.

The following clauses set out features of the invention which may not presently be claimed in this application, but which may form the basis for future amendment or a divisional application.
1. A method for capturing carbon dioxide (CO₂) from an air stream, the method comprising:
   allowing the air stream to flow through a CO₂ capturing media to capture CO₂ gas present in the air stream;
   compressing and/or liquefying the captured CO₂ gas into liquid CO₂; and
   supplying heat generated during the compression and/or liquefication of the CO₂ to the CO₂ capturing media for regeneration of the CO₂ capturing media.
2. The method of clause 1, wherein the method comprises:
   receiving, by a compressor, the captured CO₂ gas from the CO₂ capturing media and correspondingly compressing the received CO₂ gas, wherein the compression of received CO₂ gas results in heating of the compressed CO₂ gas; and
   supplying, by a conduit extending in a predefined configuration through the CO₂ capturing media, the heated compressed CO₂ gas through the CO₂ capturing media to regenerate the CO₂ capturing media and correspondingly liquefy the compressed CO₂ gas into liquid CO₂.
3. The method of clause 1, wherein the method comprises:
   receiving, by a compressor, the captured CO₂ gas from the CO₂ capturing media and correspondingly compressing the received CO₂ gas;
   allowing flow of the compressed CO₂ gas through a heat exchanger fluidically connected to an outlet of the compressor;
   allowing flow of air across the heat exchanger to facilitate heat exchange between the air and the compressed CO₂ gas, wherein the heat exchange causes the air to be heated and the compressed CO₂ gas to liquefy into liquid CO₂; and
   supplying the heated air through the CO₂ capturing media for regeneration of the CO₂ capturing media.
4. The method of any of clauses 1 to 3, wherein the method comprises the steps of supplying and storing the generated liquid CO₂ in a container.
5. The method of any of clauses 1 to 4, wherein the CO₂ capturing media is positioned in a passage or containment space that is configured to be fluidically isolated from ambient to allow the captured CO₂ to dissolve therewithin and be further collected by the compressor, wherein an inlet of the compressor is blocked during dissolving of the captured CO₂.
6. The method of any of clauses 1 to 5, wherein the method comprises the steps of monitoring, by one or more gas sensors, an amount of CO₂ captured by the CO₂ capturing media, and
   when the monitored amount of CO₂ captured by the CO₂ capturing media exceeds a predefined saturation level, the method comprises the steps of:
   receiving, by a liquefication unit, the captured CO₂ gas from the CO₂ capturing media to compress and/or liquefy the CO₂ gas; and
   supplying the heat generated during the compression and/or liquefication to the CO₂ capturing media for regeneration of CO₂ capturing media.
7. A method for capturing hydrocarbon gases from an air stream, the method comprising:
   allowing the air stream to flow through a hydrocarbon capturing media to capture hydrocarbon gases present in the air stream;
   compressing or liquefying, by a liquefication unit, the captured hydrocarbon gases into liquid hydrocarbon; and
   supplying heat generated during the compression or liquefication of the hydrocarbon gases to the hydrocarbon capturing media for regeneration of the hydrocarbon capturing media.
8. The method of clause 7, wherein the method comprises:
   receiving, by a compressor, the captured hydrocarbon gases from the hydrocarbon capturing media and correspondingly compressing the received hydrocarbon gases, wherein the compression of the hydrocarbon gases results in heating of the hydrocarbon gases; and
   supplying, by a conduit extending in a predefined configuration through the hydrocarbon capturing media, the heated compressed hydrocarbon gases through the hydrocarbon capturing media to regenerate the hydrocarbon capturing media and correspondingly liquefy the compressed hydrocarbon gases into liquid hydrocarbon.
9. The method of clause 7, wherein the method comprises:
   receiving, by a compressor, the captured hydrocarbon gases from the hydrocarbon capturing media and correspondingly compressing the received hydrocarbon gases;
   allowing flow of the compressed hydrocarbon gases through a heat exchanger fluidically connected to an outlet of the compressor;
   allowing flow of air across the heat exchanger to facilitate heat exchange between the air and the compressed hydrocarbon gases, wherein the heat exchange causes the air to be heated and the compressed hydrocarbon gases to liquefy into liquid hydrocarbon; and
   supplying the heat air through the hydrocarbon capturing media for regeneration of the hydrocarbon capturing media.
10. The method of any of clauses 7 to 9, wherein the method comprises the steps of supplying and storing the generated liquid hydrocarbon in a container, wherein the hydrocarbon gases comprise methane, propane, and/or ethane.
11. The method of any of clauses 7 to 10, wherein the method comprises the steps of monitoring, by one or more gas sensors, an amount of the hydrocarbon gases captured by the hydrocarbon capturing media, and
   when the monitored amount of hydrocarbon gases captured by the hydrocarbon capturing media exceeds a predefined saturation level, the method comprises the steps of:
   receiving, by a liquefication unit, the captured hydrocarbon gases from the hydrocarbon capturing media to compress and/or liquefy the hydrocarbon gases; and
   supplying the heat generated during the compression or liquefication to the hydrocarbon capturing media for regeneration of hydrocarbon capturing media.
12. A system for capturing hydrocarbon gases from an air stream, the system comprising:
   a hydrocarbon gas capturing media configured to allow the air stream to flow therethrough and correspondingly capture hydrocarbon gases present in the air stream; and
   a liquefication unit configured to receive the captured hydrocarbon gases from the hydrocarbon gas capturing media and correspondingly compress and/or liquefy the received hydrocarbon gases into liquid hydrocarbons,
   wherein heat generated during the compression and/or liquefication of the hydrocarbon gases is supplied to the hydrocarbon gas capturing media to regenerate the hydrocarbon gas capturing media.
13. The system of clause 12, wherein the liquefication unit comprises:
   a compressor configured to receive the captured hydrocarbon gases from the hydrocarbon gas capturing media and correspondingly compress the received hydrocarbon gases, wherein the compression of received hydrocarbon gases results in heating of the hydrocarbon gases; and
   a conduit fluidically connected to an outlet of the compressor, the conduit in thermal contact and extending in a predefined configuration through the hydrocarbon gas capturing media,
   wherein the heated compressed hydrocarbon gases are supplied through the conduit to heat the hydrocarbon gas capturing media and correspondingly regenerate the hydrocarbon gas capturing media and liquefy the compressed hydrocarbon gases into liquid hydrocarbons.
14. The system of clause 12, wherein the liquefication unit comprises:
   a compressor configured to receive the captured hydrocarbon gases from the hydrocarbon gas capturing media and correspondingly compress the received hydrocarbon gases; and
   a heat exchanger fluidically connected to an outlet of the compressor, the heat exchanger configured to allow flow of the compressed hydrocarbon gases therethrough;
   wherein the heat exchanger is further configured to allow flow of air across the heat exchanger to facilitate heat exchange between the air and the compressed hydrocarbon gases, wherein the heat exchange causes the air to heat and liquefy the compressed hydrocarbon gases into liquid hydrocarbons,
   wherein the heated air is supplied to the hydrocarbon gas capturing media to regenerate the hydrocarbon gas capturing media.
15. The system of clause 14, wherein the system comprises one or more fans to facilitate the flow of the air across the heat exchanger and further supply the heated air to the hydrocarbon gas capturing media.
16. The system of any of clauses 12 to 15, wherein the system comprises a storage container fluidically connected to the liquefication unit and configured to receive and store the generated liquid hydrocarbons.
17. The system of any of clauses 12 to 16, wherein the hydrocarbon gas capturing media is selected from a group comprising a hydrocarbon gas adsorption bed, and a hydrocarbon gas absorption bed.
18. The system of any of clauses 12 to 17, wherein the hydrocarbon gas capturing media is positioned in a passage or containment space that is configured to be fluidically isolated from ambient to allow the captured hydrocarbon gases to dissolve therewithin and be further collected by the liquefication unit, wherein an inlet of the liquefication unit is blocked during dissolving of the captured hydrocarbon gases.
19. The system of any of clauses 12 to 18, wherein the system comprises one or more gas sensors configured with the hydrocarbon gas capturing media or positioned downstream of the hydrocarbon gas capturing media, the one or more gas sensors are configured to monitor an amount of hydrocarbon gases captured by the hydrocarbon gas capturing media.
20. The system of any of clauses 12 to 19, wherein when the monitored amount of hydrocarbon gases captured by the hydrocarbon gas capturing media exceeds a predefined saturation level, the system is configured to actuate the liquefication unit to:
   receive the captured hydrocarbon gases from the hydrocarbon gas capturing media to compress or liquefy the hydrocarbon gases; and
   supply the heat generated during the compression or liquefication to the hydrocarbon gas capturing media for regeneration of hydrocarbon gas capturing media.
21. The system of any of clauses 12 to 20, wherein the hydrocarbon gases comprise methane, propane, and/or ethane.

## Claims

1. A system (100) for capturing carbon dioxide (CO₂) from an air stream, the system comprising:
a CO₂ capturing media (102) configured to allow the air stream to flow therethrough and correspondingly capture CO₂ gas present in the air stream; and
a liquefication unit configured to receive the captured CO₂ gas from the CO₂ capturing media and correspondingly compress and/or liquefy the received CO₂ gas into liquid CO₂,
wherein heat generated during the compression and/or liquefication of the CO₂ is supplied to the CO₂ capturing media to regenerate the CO₂ capturing media.

2. The system of claim 1, wherein the liquefication unit comprises:
a compressor (104) configured to receive the captured CO₂ gas from the CO₂ capturing media (102) and correspondingly compress the received CO₂ gas, wherein the compression of received CO₂ gas results in heating of the CO₂ gas; and
a conduit (106) fluidically connected to an outlet of the compressor (104-2), the conduit in thermal contact and extending in a predefined configuration through the CO₂ capturing media (102),
wherein the heated compressed CO₂ gas is supplied through the conduit (106) to heat the CO₂ capturing media (102) and correspondingly regenerate the CO₂ capturing media (102) and liquefy the compressed CO₂ gas into liquid CO₂.

3. The system of claim 1, wherein the liquefication unit comprises:
a compressor (104) configured to receive the captured CO₂ gas from the CO₂ capturing media (102) and correspondingly compress the received CO₂ gas; and
a heat exchanger (202) fluidically connected to an outlet of the compressor (104), the heat exchanger (202) configured to allow flow of the compressed CO₂ gas therethrough;
wherein the heat exchanger (202) is further configured to allow flow of air across the heat exchanger (202) to facilitate heat exchange between the air and the compressed CO₂ gas, wherein the heat exchange causes the air to heat and liquefy the compressed CO₂ gas into liquid CO₂,
wherein the heated air is supplied to the CO₂ capturing media (102) to regenerate the CO₂ capturing media (102).

4. The system of claim 3, wherein the system (100) comprises one or more fans (204) to facilitate the flow of the air across the heat exchanger (202) and further supply the heated air to the CO₂ capturing media (102).

5. The system of any of claims 1 to 4, wherein the system (100) comprises a storage container (108) fluidically connected to the liquefication unit and configured to receive and store the generated liquid CO₂.

6. The system of any of claims 1 to 5, wherein the CO₂ capturing media (102) is selected from a group comprising a CO₂ adsorption bed, and a CO₂ absorption bed.

7. The system of any of claims 1 to 6, wherein the CO₂ capturing media (102) is positioned in a passage or containment space that is configured to be fluidically isolated from ambient to allow the captured CO₂ to dissolve therewithin and be further collected by the liquefication unit, wherein an inlet of the liquefication unit is blocked during dissolving of the captured CO₂.

8. The system of any of claims 1 to 7, wherein the system (100) comprises one or more gas sensors (110) configured with the CO₂ capturing media or positioned downstream of the CO₂ capturing media, wherein the one or more gas sensors (110) are configured to monitor an amount of CO₂ captured by the CO₂ capturing media (102).

9. The system of any of claims 1 to 8, wherein when the monitored amount of CO₂ captured by the CO₂ capturing media (102) exceeds a predefined saturation level, the system is configured to actuate the liquefication unit to:
receive the captured CO₂ gas from the CO₂ capturing media (102) to compress or liquefy the CO₂ gas; and
supply the heat generated during the compression or liquefication to the CO₂ capturing media (102) for regeneration of CO₂ capturing media (102).

10. A method (300, 400) for capturing a gas from an air stream, wherein the gas is carbon dioxide (CO₂) or hydrocarbon gases, the method comprising:
allowing the air stream to flow through a gas capturing media (102) to capture the gas present in the air stream;
compressing and/or liquefying the captured gas into liquid; and
supplying heat generated during the compression and/or liquefication of the gas to the gas capturing media (102) for regeneration of the gas capturing media.

11. The method of claim 10, wherein the method (300, 400) comprises:
receiving, by a compressor (104), the captured gas from the gas capturing media (102) and correspondingly compressing the received gas, wherein the compression of received gas results in heating of the compressed gas; and
supplying, by a conduit extending in a predefined configuration through the gas capturing media (102), the heated compressed gas through the gas capturing media (102) to regenerate the gas capturing media (102) and correspondingly liquefy the compressed gas into liquid.

12. The method of claim 10, wherein the method comprises:
receiving, by a compressor (104), the captured gas from the gas capturing media (102) and correspondingly compressing the received gas;
allowing flow of the compressed gas through a heat exchanger fluidically connected to an outlet of the compressor (104);
allowing flow of air across the heat exchanger (202) to facilitate heat exchange between the air and the compressed gas, wherein the heat exchange causes the air to be heated and the compressed gas to liquefy into liquid; and
supplying the heated air through the gas capturing media (102) for regeneration of the gas capturing media.

13. The method of any of claims 10 to 12, wherein the method (300, 400) comprises the steps of supplying and storing the generated liquid in a container.

14. The method of any of claims 10 to 13, wherein the gas is CO₂ and CO₂ capturing media (102) is positioned in a passage or containment space that is configured to be fluidically isolated from ambient to allow the captured CO₂ to dissolve therewithin and be further collected by the compressor (104), wherein an inlet of the compressor (104-1) is blocked during dissolving of the captured CO₂,
or wherein the gas comprises hydrocarbon gases comprising methane, propane, and/or ethane.

15. The method of any one of claims 10 to 14, wherein the method comprises the steps of monitoring, by one or more gas sensors (110), an amount of gas captured by the gas capturing media (102), and
when the monitored amount of gas captured by the gas capturing media (102) exceeds a predefined saturation level, the method comprises the steps of:
receiving, by a liquefication unit, the captured gas from the gas capturing media (102) to compress and/or liquefy the gas; and
supplying the heat generated during the compression and/or liquefication to the gas capturing media for regeneration of gas capturing media (102).
